# EUROPEAN PATENT APPLICATION

(11) **EP 4 292 569 A1**
(43) Date of publication of application: **20.12.2023**
(21) Application number: 21945698.5
(22) Date of filing: 25.08.2021
(51) Int. Cl.: A61F 2/24

(54) **ANCHORING DEVICE FOR HEART VALVE**

(30) Priority: 17.06.2021 CN 202110673344; 17.06.2021 CN 202121346726 U
(71) Applicant: Shanghai Trulive Medtech Co., Ltd, Shanghai 201206 (CN); Jiangsu Trulive Medtech Co., Ltd, Rudong County Nantong Jiangsu 226499 (CN)
(72) Inventor: HE, Dong, Shanghai 201206 (CN); ZHANG, Haijun, Shanghai 201206 (CN); LIU, Xiang, Shanghai 201206 (CN); ZHAO, Jing, Shanghai 201206 (CN)
(74) Representative: Nordmeyer, Philipp Werner
(86) International application number: PCT/CN2021/114559
(87) International publication number: WO 2022/262117

(57) **Abstract**

An anchoring device for a cardiac valve is disclosed. The anchoring device includes a ventricular section (1), the ventricular section (1) includes an anchoring segment (11) and a capture segment (12), the anchoring segment (11) is in the form of a helical coil, a radius of curvature of the helical coil is constant and a distance between turns of the helical coil is the same, and the anchoring device does not include an atrial section. The anchoring device is made of an elastic material. The design of the anchoring device separating from the valve can provide an anchoring effect while allowing a delivery sheath of an associated delivery system to have a reduced radial dimension. During release of the anchoring device, the capture segment (12) can automatically capture the native leaflets or chordae tendineae. It is a modification of an existing anchor ring and dispenses with the use of a guide wire. The anchoring segment (11) can automatically coil around the native leaflets, thereby simplifying an associated delivery device and step. Moreover, the anchoring effect is good, and self-adaptation to the shape of a prosthetic valve can be achieved.

## Description

### TECHNICAL FIELD

The present invention relates to the field of medical devices and, in particular, to an anchoring device for a cardiac valve.

### BACKGROUND

The heart has four chambers: the right atrium (RA), the right ventricle (RV), the left atrium (LA) and the left ventricle (LV). The pumping action of the left and right sides of the heart occurs generally in synchrony during the overall cardiac cycle. The atria are separated from the ventricles by the so-called atrioventricular valves, which are one-way valves ensuring normal flow of blood in the heart. The atrioventricular valve separating the left atrium from the left ventricle is the so-called mitral valve, and the atrioventricular valve separating the right atrium from the right ventricle is the so-called tricuspid valve. The pulmonary valve directs blood flow to the pulmonary artery and thence to the lungs; blood returns to the left atrium via the pulmonary veins. The aortic valve directs flow through the aorta and thence to the periphery. There are normally no direct connections between the ventricles or between the atria.

At the beginning of ventricular filling (diastole), the aortic and pulmonary valves are closed to prevent back flow from the arteries into the ventricles. Shortly thereafter, the atrioventricular valves open to allow unimpeded flow from the atria into the corresponding ventricles. Shortly after ventricular systole (i.e., ventricular emptying) begins, the tricuspid and mitral valves normally shut, preventing flow from the ventricles back into the corresponding atria.

A damaged atrioventricular valve may fail to function properly, resulting in improper closing. The atrioventricular valves are complex structures that generally include an annulus, leaflets, chordae and a support structure. Each atrium interfaces with its valve via an atrial vestibule. The mitral valve has two leaflets, and opposition or engagement of corresponding surfaces of leaflets against each other helps provide closure or sealing of the valve to prevent blood flowing in the wrong direction. Failure of the leaflets to seal during ventricular systole is known as malcoaptation and may allow blood to flow backward through the valve (regurgitation). Heart valve regurgitation can have serious consequences to a patient, often resulting in cardiac failure, decreased blood flow, lower blood pressure, and/or a diminished flow of oxygen to the tissues of the body. Mitral regurgitation can also cause blood to flow back from the left atrium to the pulmonary veins, causing congestion. Severe valvular regurgitation, if untreated, can result in permanent disability or death.

Transcatheter mitral valve replacement (TMVR) is an interventional technique to deliver a prosthetic valve, which has been crimped into a delivery system outside the body of a patient, with a catheter to the native mitral valve annulus through a vascular or transapical access and release the prosthetic valve at the native mitral valve annulus so that the it can function in place of the native one. Compared with the surgical operation, TMVR dispenses with the aid of a cardiopulmonary bypass machine, causes less trauma, and allows faster patient recovery and significant improvements in postoperative hemodynamic indices. Compared with transapical delivery approach, the transseptal approach causes even less trauma and is suitable for a wider range of patients.

Although mitral valve replacement technology has advanced rapidly, it is facing with a number of well-recognized challenges in terms of valve design, such as valve anchoring. Most conventional anchor designs rely on anchor tines which capture the native leaflet or an oversized valve body (the term "oversized" refers to a design size of the prosthetic valve greater than that of the native tissue, which allows the prosthetic valve, when released, to press against and anchor to the native tissue). However, these approaches tend to damage the native leaflets or push on the tissue of the native annulus, adversely affecting patient recovery.

### SUMMARY OF THE INVENTION

In view of the above discussed shortcomings of the prior art, it is an objective of the present invention to provides an anchoring device for a cardiac valve, which can overcome the problems with the prior art.

In order to achieve the above and other objectives, the present invention provides an anchoring device for a cardiac valve, which includes a ventricular section including an anchoring segment in the form of a helical coil having a constant radius of curvature and a uniform turn-to-turn spacing. The anchoring device does not include an atrial section.

Preferably, the anchoring device may be made of an elastic material.

Preferably, a capture segment in the form of a helical coil may extend from one end of the anchoring segment.

Preferably, the coil of the capture segment may have 0.5-3 turns.

Preferably, the capture segment may have a radius of curvature greater than that of the anchoring segment.

The present invention also provides a cardiac valve replacement apparatus including the anchoring device and a prosthetic valve adapted to be entirely or partially received in an internal space defined by the anchoring device.

The present invention also provides use of the anchoring device and/or the cardiac valve replacement apparatus in manufacture of a cardiac valve replacement product.

The present invention also provides a cardiac valve replacement system including the cardiac valve replacement apparatus and a delivery system.

Preferably, the delivery system includes a delivery sheath, a delivery catheter and a connecting member. The delivery catheter nests in the delivery sheath, and the connecting member is connected to the delivery catheter.

Preferably, the connecting member is a connecting wire or a connector.

As noted above, the anchoring device of the present invention has the benefits as follows:
1. The anchoring device, when released, can capture the native leaflets or chordae tendineae by itself.
2. The anchoring device is separate from the valve. This design can provide an anchoring effect while allowing a delivery sheath of an associated delivery system to have a reduced radial dimension.
3. It is a modification of an existing anchor ring and dispenses with the use of a guide wire. It can coil around the native leaflets by itself, thereby simplifying an associated delivery device and step.
4. The anchoring effect is good, and self-adaptation to the shape of a prosthetic valve can be achieved.

### BRIEF DESCRIPTION OF THE DRAWINGS

Fig. 1 schematically illustrates an anchoring device for a cardiac valve according to the present invention.
Fig. 2 schematically illustrates a prosthetic valve and an implantation process thereof according to the present invention.
Fig. 3 shows a schematic cross-sectional view of an implanted cardiac valve replacement apparatus according to the present invention.
Fig. 4 schematically illustrates a connecting member according to an embodiment of the present invention.
Fig. 5 schematically illustrates a connecting member according to another embodiment of the present invention.
Fig. 6 schematically illustrates an implantation process of an anchoring device for a cardiac valve according to the present invention.

### List of Reference Numerals

1 Ventricular Section
11 Anchoring Segment
111 Free End of Anchoring Segment
111a Through Slot
12 Capture Segment
121 Free End of Capture Segment
2 Prosthetic Valve
21 Outflow Section
22 Annulus Section
23 Inflow Section
31 Delivery Sheath
32 Delivery Catheter
33 Connecting Member
331 Threaded End
332 Smooth End
41 Aorta
42 Native Leaflets
43 Chordae Tendineae
H Height

### DETAILED DESCRIPTION

Embodiments of present invention will be described below by way of specific examples. Other advantages and benefits of the present invention can be readily appreciated by those familiar with the art from the disclosure herein.

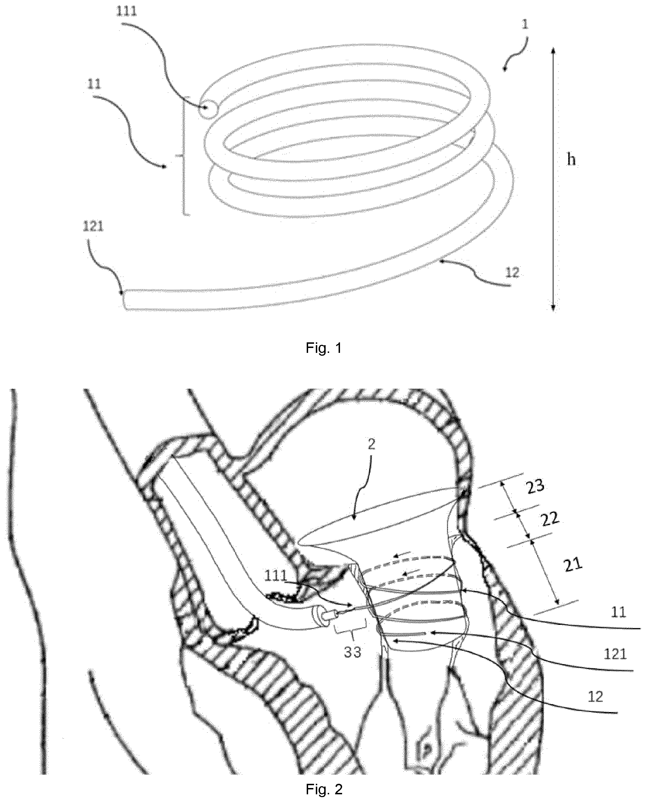

Reference is now made to Figs. 1 to 6. It should be noted that structural, proportional, dimensional and other details in the figures are presented only for the purpose of facilitating, in conjunction with the disclosure herein, the understanding and reading of those familiar with the art rather than being intended to limit conditions under which the present invention can be implemented. Therefore, they are technically of no substantive significance, and any and all structural modifications, proportional variations or dimensional changes that do not affect the benefits and objects of the present invention are considered to fall within the scope of the teachings herein. Moreover, the terms "upper", "lower", "left", "right", "middle", "a", "an", "the" and the like are used herein merely for the sake of ease of explanation, rather than to limit the scope of implementation of the invention in any way. Any and all changes or modifications to relative relationships, as long as they do not alter the essence of the invention, are considered to fall within its scope of implementation.

As shown in Fig. 1, the present invention provides an anchoring device for a cardiac valve. The anchoring device includes a ventricular section 1. The ventricular section 1 includes an anchoring segment 11. The anchoring segment 11 is in the form of a helical coil with a constant radius of curvature and a uniform turn-to-turn spacing. The anchoring device does not include an atrial section.

According to the present invention, the anchoring device may also be referred to as an anchoring coil.

The anchoring device is made of an elastic material such as an elastic wire. In a preferred embodiment, the anchoring device is formed of one or more elastic wires. The elastic wires can greatly elastically deform, either axially, or radially and circumferentially. The anchoring device has resilience determined by its material and shape.

The elastic wires are made of a material selected from a metallic material and/or a polymeric material. The metallic material is selected from one or more of the following: stainless steel, titanium and alloys thereof, and nitinol. The polymeric material is, for example, nylon. Each of the elastic wires may be made up of two or more sections of different ones of the materials enumerated above.

Each elastic wire may have a circular or rectangular cross-section. Each elastic wire may have a constant cross-sectional area, i.e., a uniform thickness or thinness. Alternatively, it may have a varying cross-sectional area.

In one embodiment, each elastic wire is provided with a cover layer on its surface. In one embodiment, the cover layer is a coating or jacket. The coating may be formed by, for example, spraying an organic material. The jacket is a hollow tube, which can be disposed over the elastic wire. The hollow tube may be an extruded seamless tube or rolled seamed tube. The cover layer may be made of polyethylene, polytetrafluoroethylenes, polyethylene terephthalate (PET) or other materials, or a combination thereof.

The coil of the anchoring segment 11 has at least one turn. In a preferred embodiment, the coil of the anchoring segment 11 has 3 turns.

Each turn in the helical coil of the anchoring segment 11 has a shape selected from an arc-like shape, a circular shape and an elliptic shape. Each turn in the helical coil of the anchoring segment 11 has a radius of curvature of 5-20 mm.

Said turn-to-turn spacing of the helical coil refers to a distance between adjacent coil turns.

The anchoring segment 11 functions mainly to provide a force required by anchoring of the prosthetic valve.

In one embodiment, the anchoring segment 11 is provided with a cover layer on its surface, which can enhance the anchoring because of having a higher coefficient of friction. Greater friction is more conducive to the anchoring.

In one embodiment, a capture segment 12 extends from one end of the anchoring segment 11. The capture segment 12 is in the form of a helical coil.

The coil of the capture segment 12 has 0.5-3 turns. The capture segment 12 may consist of a single or multiple arcs, each having a radius of curvature of 5-30 mm.

The capture segment 12 functions principally to capture native tissue such as the leaflets and chordae tendineae to provide guidance for the subsequent positioning and anchoring of the anchoring segment 11.

In one embodiment, the capture segment 12 is provided with a cover layer on its surface.

The cover layer can enhance the capture because of having a lower coefficient of friction. Less friction can reduce resistance to the capture segment 12 during its travel.

The capture segment 12 is joined to said end of the anchoring segment 11 so that each of the capture segment 12 and the anchoring segment 11 has a respective free end. That is, the capture segment has a free end 121, and the anchoring segment has a free end 111.

The free end 111 of the anchoring segment is coupled to a delivery system. Depending on the different delivery system used to implant the anchoring device into the body of a patient, various features may be provided at the free end 111 of the anchoring segment, such as a thread or through slot 111a. The present invention is not limited to any particular shape of the through slot 111a. For example, it may have a circular, rectangular, waist or otherwise-shaped cross-section. An axis of the through slot 111a may be perpendicular or parallel to a plane defined by the anchoring device. With this configuration, the anchoring device can be securely coupled to the delivery system without falling off of the anchoring device during implantation.

It is to be noted that during or after release of the anchoring device, movement of the native valve may be restricted to a certain extent, but it still performs the basic functions. Therefore, the heart can function normally during surgery, dispensing with the need to use an extracorporeal membrane oxygenator or stop the heart. This gives a physician more time to perform subsequent preliminary operations for prosthetic valve implantation or surgical operations.

A turn-to-turn spacing between the capture segment 12 and the anchoring segment 11 may be the same as or different from that of the anchoring segment 11, as long as a suitable height h is ensured for the anchoring device of the cardiac valve, which may range from 3 mm to 20 mm.

The radius of curvature of the capture segment 12 is greater than that of the anchoring segment 11. The greater radius of curvature of the capture segment 12 can facilitate capture of the native leaflets or chordae tendineae. The smaller radius of curvature of the anchoring segment 11 enables to provide a radial force required by the anchoring.

Anchoring devices of the prior art have an atrial section, which is positioned in an atrium after the anchoring device is implanted into the body of a patient. This atrial section will compress the annulus and tends to cause damage to the native leaflets, thus adversely affecting patient recovery. In contrast, the anchoring device of this application has only the ventricular section but not an atrial section. Therefore, it will not compress the annulus and is prevented from falling off.

The present invention also provides a cardiac valve replacement apparatus including an anchoring device as defined above and a prosthetic valve 2. The prosthetic valve 2 is adapted to be partially or entirely received in an internal space defined by the anchoring device.

As shown in Fig. 2, the prosthetic valve 2 is in the shape of a tube open at both ends. It includes an outflow section 21 and an annulus section 22, which are joined together. The outflow section 21 is adapted to be entirely or partially received in the internal space defined by the anchoring device. Alternatively or additionally, the annulus section 22 is adapted to be entirely or partially received in the internal space defined by the anchoring device.

In a preferred embodiment, the prosthetic valve 2 further includes an inflow section 23 joined to the annulus section 22. The inflow section 23 is configured as a flange, which can stabilize the prosthetic valve 2 and prevent perivalvular leak.

The tube is hollow and may be in the form of, for example, a cylindrical, tapered, waist or otherwise-shaped tube.

The prosthetic valve 2 can replace the native mitral or tricuspid valve. It may be delivered into the atrium through the inferior vena cava or another path and then through the atrial septum into the internal space defined by the anchoring device. Subsequently, it may be released into the anchoring device, in one pass from an expanded balloon, from a sheath in a self-expanding and step-by-step manner, or otherwise.

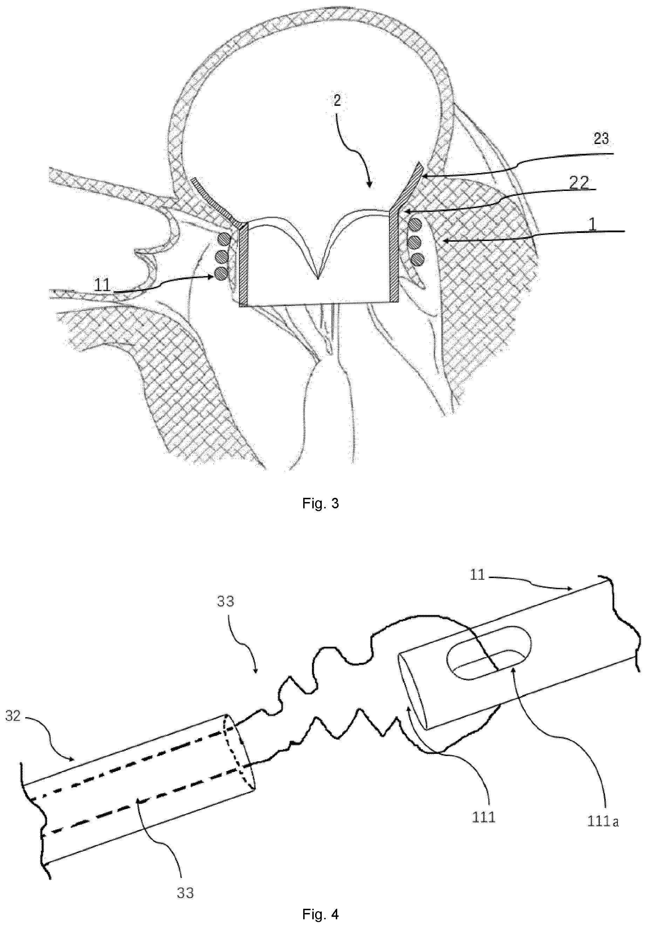

Fig. 3 shows a cross-sectional view of the cardiac valve replacement apparatus that has been implanted into the body of a patient. As shown, the prosthetic valve 2 has the inflow section 23 and is implanted at the mitral valve, and the outflow section 21 is generally cylindrical. The annulus section 22 is seated at the native annulus so that, under the action of the anchoring device and the prosthetic valve 2, the native tissue (leaflets or chordae tendineae) fits the contour of the prosthetic valve 2, with the anchoring device uniformly surrounding the native leaflets or chordae tendineae and providing anchoring support. Both the free end 121 of the capture segment and the free end 111 of the anchoring segment fit the native leaflets or valve, reducing the influence on blood flow within the heart.

The present invention also provides use of the anchoring device and/or the cardiac valve replacement apparatus in the manufacture of a cardiac valve replacement product.

In the cardiac valve replacement product, the anchoring device and the prosthetic valve 2 are delivered through different paths.

The present invention also provides a cardiac valve replacement system including the cardiac valve replacement apparatus as defined above and a delivery system.

The delivery system is used to implant the cardiac valve replacement apparatus into the body of a patient.

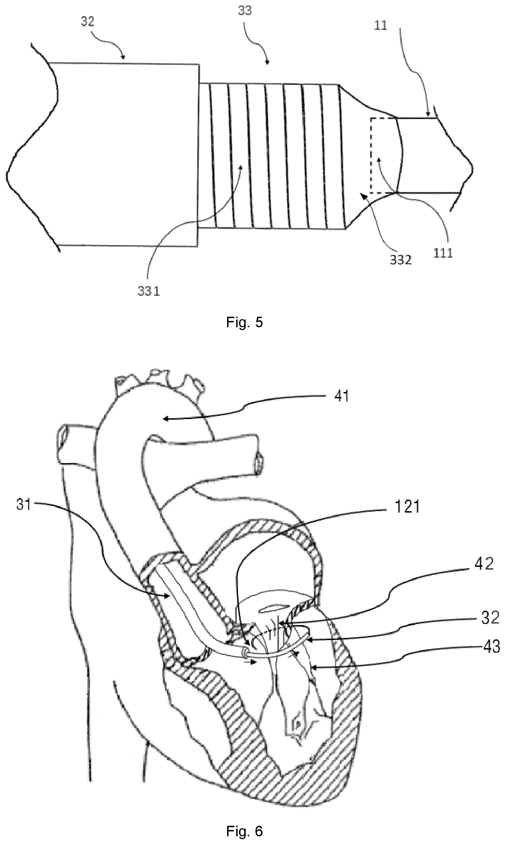

As shown in Figs. 2 and 6, the delivery system includes a delivery sheath 31, a delivery catheter 32 and a connecting member 33. The delivery catheter 32 nests within the delivery sheath 31, and the connecting member 33 connects the delivery catheter 32. The delivery system may further include other components, such as a handle.

In the embodiment shown in Fig. 4, the connecting member 33 is a connecting wire selected from a metallic elastic wire, a surgical suture, a tether, a strip and the like. The connecting wire may be made of one or more materials, such as one or more of nickel-titanium alloys, stainless steel, nylon and other biochemical materials. One or more such connecting wires may be included. In this embodiment, a through slot 111a may be provided at the free end 111 of the anchoring segment. The connecting wire may be passed through the through slot 111a and the delivery catheter 32 and then tied to the handle or another component of the delivery system. It may be otherwise secured to the through slot 111a. Preferably, the connecting wire may have a margin within or out of the delivery catheter 32, which allows the free end 111 of the anchoring segment to freely retract until it fits the valve contour when the anchoring device uncoils. After the prosthetic valve 2 is implanted, the connecting wire is cut and withdrawn from the delivery system.

In the embodiment shown in Fig. 5, the connecting member 33 is a connector having a threaded end 331 and an opposite, smooth end 332. In this embodiment, the delivery catheter 32 has a thread matching that of the threaded end 331. During implantation, the smooth end 332 of the connector is fixedly connected to the free end 111 of the anchoring segment. The fixed connection is accomplished by a method selected from welding and clamping.

The cardiac valve of the present invention is selected from a mitral valve and a tricuspid valve.

The cardiac valve replacement system of the present invention is implanted in the manner described below.

The anchoring device is released as shown in Fig. 2. The delivery sheath 31 may be advanced into the left ventricle through the aorta 41 or another path, and the delivery catheter 32 may be then released from the delivery sheath 31. The delivery catheter 32 may extend on the ventricular wall into a semicircular or otherwise curved shape which surrounds part of the native tissue (leaflets and/or chordae tendineae). Thus, it provides a path for release of the anchoring device. After the path is established, the anchoring device may be advanced through the delivery catheter 32 into the left ventricle. The free end 121 of the capture segment may be first released, due to the relatively large radius of curvature, it can also follow the path established on the ventricular wall to capture the native leaflets 42 and/or chordae tendineae 43 and form coils substantially in parallel to the annulus. The remainder of the anchoring device may be further released following the path established by the capture segment. As the anchoring device advances, the diametrically smaller anchoring segment 11 begins to enter the ventricle to additionally surround the native leaflets 42 and/or chordae tendineae 43 within the anchoring device. The anchoring device may be further advanced until the free end 111 of the anchoring segment leaves the delivery catheter 32, thus completing the release of the anchoring device.

The prosthetic valve is implanted in the manner shown in Fig. 3. After the release of the anchoring device is complete, the implantation of the prosthetic valve 2 may be initiated. The prosthetic valve 2 may be advanced into the atrium through the inferior vena cava or another path and then into the anchoring device through the atrial septum. After that, it may be released within the anchoring device, either in one pass from an expanded balloon, or from a sheath in a self-expanding and step-by-step manner. Under the action of a force, the anchoring segment 11 may be expanded in diameter, resulting in elastic and/or plastic deformation of the anchoring device. During the deformation of the anchoring segment 11, it will fit into the contour of the prosthetic valve 2, providing anchoring support to the prosthetic valve 2. As a result of the diametrical expansion of the anchoring segment 11, the capture segment 12 will uncoil, leading to retraction of the free end 121 of the capture segment along the coil. Preferably, the capture segment 12 closely fits the contour of the prosthetic valve 2 to provide an anchoring effect, even after the uncoiling. During the deformation of the anchoring segment 11, the free end 111 of the anchoring segment may also tend to uncoil. In this case, the uncoiling of the free end 111 of the anchoring segment may be controlled by the connecting member 33 to avoid blood from flowing out from the ventricle. Additionally, after the uncoiling of the free end 111 of the anchoring segment, it may still closely fit the contour of the prosthetic valve 2 and provide an anchoring effect. After the prosthetic valve 2 is completely released, if it is confirmed that the prosthetic valve 2 and the anchoring device have been firmly anchored at the desired locations, the delivery system may be withdrawn, and the connecting member 33 may be detached from the free end 111 of the anchoring segment in the anchoring device. After the delivery system is withdrawn, the implantation of the prosthetic valve 2 and the anchoring device ends. Fig. 3 shows a cross-sectional view of the cardiac valve replacement apparatus that has been implanted into the body of a patient.

According to the present invention, the anchoring device is implanted through the aorta, while the prosthetic valve is implanted through another path. This allows a delivery system for the anchoring device not to be withdrawn during the implantation of the prosthetic valve. The anchoring device can be held stationary with the delivery system until the prosthetic valve has been deployed at the desired location. In this way, the anchoring device of the present invention is allowed to not have an atrial section, thus exerting less compression on cardiac tissue and encountering reduced resistance. Moreover, the implantation through the aorta rather than the atrial septum allows the anchoring device to be initially released within the ventricle. Moreover, it can be expanded in diameter as a result of the subsequent release of the prosthetic valve to provide an anchoring effect. In contrast, conventional anchoring devices and prosthetic valves are both implanted through the cardiac apex or the atrial septum. Accordingly, a delivery system for the anchoring device must be withdrawn before the prosthetic valve can be implanted. However, after the delivery system is withdrawn, the anchoring device will easily fall off if it does not have an atrial section for securing. For this reason, conventional anchoring device must have such an atrial section which can secure the anchoring device and ensure that it will not fall off after the delivery system is withdrawn during the implantation of the prosthetic valve.

In summary, the present invention has effectively overcome the various drawbacks of the prior art and has a high value in industrial use.

The embodiments disclosed hereinabove are solely for the purpose of exemplary illustration of the principles and benefits of the present invention and not for the purpose of limiting the invention. Any person familiar with the art can make modifications or changes to the disclosed embodiments without departing from the spirit and scope of this invention. Accordingly, any and all equivalent modifications or changes made by any person with general common knowledge in the art without departing from the spirit and teachings of the present application are intended to be embraced within the scope as defined by the appended claims.

## Claims

1. An anchoring device for a cardiac valve, the anchoring device comprising a ventricular section (1), the ventricular section (1) comprising an anchoring segment (11), the anchoring segment (11) in the form of a helical coil, wherein a radius of curvature of the helical coil is constant and a distance between turns of the helical coil is the same, and the anchoring device does not comprise an atrial section.

2. The anchoring device according to claim 1, wherein the anchoring device is made of an elastic material.

3. The anchoring device according to claim 2, wherein a surface of the elastic material is provided with a cover layer.

4. The anchoring device according to claim 1, wherein a capture segment (12) extends from one end of the anchoring segment (11), and the capture segment (12) is in the form of a helical coil.

5. The anchoring device according to claim 4, wherein the coil of the capture segment (12) has 0.5-3 turns.

6. The anchoring device according to claim 1, wherein the anchoring device has a height h of 3-20 mm.

7. The anchoring device according to claim 4, wherein the capture segment (12) has a radius of curvature greater than that of the anchoring segment (11).

8. A cardiac valve replacement apparatus, comprising the anchoring device of any one of claims 1 to 7 and a prosthetic valve (2), the prosthetic valve (2) is adapted to be entirely or partially received in an internal space defined by the anchoring device.

9. The cardiac valve replacement apparatus according to claim 8, wherein the prosthetic valve (2) is a tube open at both ends and comprises an outflow section (21), an annulus section (22) and an inflow section (23), which are joined together sequentially, wherein the outflow section (21) is adapted to be entirely or partially received in the internal space defined by the anchoring device, and/or wherein the annulus section (22) is adapted to be entirely or partially received in the internal space defined by the anchoring device.

10. Use of the anchoring device of any one of claims 1 to 7 and/or the cardiac valve replacement apparatus of any one of claims 8 to 9 in manufacture of a cardiac valve replacement product.

11. The use according to claim 10, wherein the anchoring device and the prosthetic valve (2) in the cardiac valve replacement product are implanted through different paths.

12. A cardiac valve replacement system, comprising the cardiac valve replacement apparatus of any one of claims 8 to 9 and a delivery system.

13. The cardiac valve replacement system according to claim 12, wherein the delivery system comprises a delivery sheath (31), a delivery catheter (32) and a connecting member (33), the delivery catheter (32) nesting in the delivery sheath (31), the connecting member (33) connected to the delivery catheter (32).

14. The cardiac valve replacement system according to claim 13, wherein the connecting member (33) is a connecting wire, wherein a free end (111) of the anchoring segment is correspondingly provided with a through slot (111a).

15. The cardiac valve replacement system according to claim 13, wherein the connecting member (33) is a connector, one end of the connector is a threaded end (331) and the other end of the connector is a smooth end (332), correspondingly, one end of the delivery catheter (32) is provided with a thread matching the threaded end (331), and wherein the smooth end (332) is adapted to be connected to a free end (111) of the anchoring segment.
